(19) Europäisches Patentamt
European Patent Office
Office européen des brevets

(11) Publication number: **0 038 223**
**B1**

(12) **EUROPEAN PATENT SPECIFICATION**

(45) Date of publication of patent specification: **08.08.84**

(51) Int. Cl.$^3$: **C 07 C 63/70,**
C 07 C 51/62,
C 07 C 51/58, C 07 B 9/00

(21) Application number: **81301678.9**

(22) Date of filing: **15.04.81**

(54) **Process for the preparation of trifluoromethylbenzoyl halides.**

(30) Priority: **16.04.80 US 140893**
**16.04.80 US 140894**

(43) Date of publication of application:
**21.10.81 Bulletin 81/42**

(45) Publication of the grant of the patent:
**08.08.84 Bulletin 84/32**

(84) Designated Contracting States:
**BE CH DE FR GB IT LI NL**

(56) References cited:
EP-A-0 018 481
DE-A-1 965 782
DE-B-2 356 257
FR-A- 820 696
FR-A-1 452 159
GB-A-1 059 747
GB-A-1 556 566
US-A-2 181 554

(73) Proprietor: **OCCIDENTAL CHEMICAL CORPORATION**
**P.O. Box 189**
**Niagara Falls New York 14302 (US)**

(72) Inventor: **Cotter, Byron R.**
**262 Laurie Lane**
**Grand Island New York 14072 (US)**
Inventor: **Tang, David Y.**
**88 Parkhaven Drive**
**Tonawanda New York, 14150 (US)**

(74) Representative: **Myerscough, Philip Boyd et al,**
**J.A. Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 038 223 B1**

Courier Press, Leamington Spa, England.

**Description**

Background of the invention

This invention relates to a process for the preparation of trifluoromethylbenzoyl chlorides and trifluoromethylbenzoyl bromides. The products are useful intermediates for the production of various dyestuffs and agricultural chemical products.

Various methods are known for the preparation of trifluoromethylbenzoyl halides. It is known, for example, to prepare trifluoromethylbenzoyl chloride by reaction of trifluoromethylbenzoic acid with thionyl chloride. The starting material for such reaction, trifluoromethylbenzoic acid may be prepared by several methods, each of which is a multi-stepped process involving relatively expensive starting materials and/or intermediates. Thus, for example, it is known to prepare trifluoromethylbenzoic acid by cyanation of bromo-benzotrifluoride and hydrolysis of resulting cyanobenzotrifluoride. In another known method, bromo-benzotrifluoride is reacted with magnesium and the resultant Grignard reagent is reacted with carbon dioxide to form trifluoromethylbenzoic acid.

In still another method xylene may be oxidized to toluic acid, the acid group esterified, and the methyl group chlorinated and then fluorinated to yield trifluoromethylbenzoic acid.

Although the processes of the prior art are useful for the preparation of trifluoromethylbenzoyl chloride, it will be appreciated that improvements in the efficiency, economy and preparation and yield of the desired product are nevertheless desirable.

The present invention provides a simple, direct process for the conversion of trihalomethylbenzoyl halide mixtures to trifluoromethylbenzoyl chlorides or bromides.

According to the invention there is provided a process for the preparation of trifluoromethylbenzoyl halides of the formula

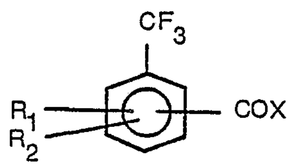

(I)

wherein X is chlorine or bromine and $R_1$ and $R_2$ independently represent hydrogen, fluorine, chlorine, bromine, alkyl, aryl, haloaryl, alkoxy, fluoroalkoxy, aryloxy, haloaryloxy, nitro, cyano, sulfonyl or carboxylic acid chloride, characterised by reacting a compound of the formula

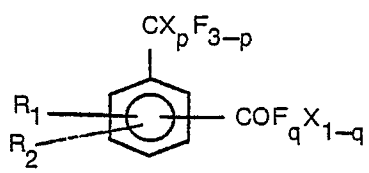

(II)

wherein p is 1, 2 or 3, q is 0 or 1 and X, $R_1$ and $R_2$ are as defined herein, with a fluorinating agent selected from hydrogen fluoride and a compound of the formula

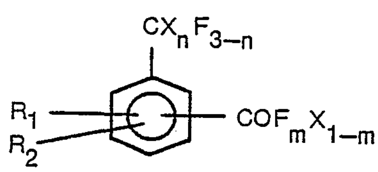

(III)

wherein n is 0, 1 or 2, m is 0 or 1, provided that when n is 0 m is 1, and X, $R_1$ and $R_2$ are as defined herein, said fluorinating agent being provided in an amount sufficient to replace the chlorine or bromine atom(s) represented by $X_p$ of formula (II) by fluorine, in the presence of a halogen transfer catalyst.

The preferred alkyl, alkoxy and fluoroalkyl groups represented by $R_1$ and $R_2$ are those of one to six carbon atoms, and most preferably methyl, methoxy, and fluoromethoxy groups. The preferred aryl and aryloxy groups are phenyl phenoxy or substituted phenyl and phenoxy groups wherein substituents such as chloro-, fluoro-, bromo-, nitro-, cyano-, methyl-, or carboxylic acid chloride are present on the ring. The most preferred trihalobenzoyl halide reactants are those of the above formula wherein $R_2$ is hydrogen and $R_1$ is hydrogen, fluorine, chlorine, bromine, nitro, cyano, or methyl.

In addition to the $R_1$ and $R_2$ substituents identified hereinabove, various other ring substituents which are stable and inert under the reaction conditions of the process of the invention, may be present. Thus, in accordance with the process of this invention, substituted or unsubstituted trichloromethylbenzoyl chlorides, dichlorofluoromethylbenzoyl chlorides, chlorodifluoromethylbenzoyl chlorides, tribromomethylbenzoyl bromides, dibromofluoromethylbenzoyl bromides, and bromodifluoromethyl-

benzoyl bromides may be reacted with the fluorinating agents defined in the presence of a halogen transfer catalyst to produce the corresponding substituted or unsubstituted trifluoromethylbenzoyl chloride or bromide.

In the halogen exchange process of the invention, the hydrogen fluoride is reacted with the trihalomethylbenzoyl chloride or bromide in an amount sufficient to replace the chlorine or bromine atoms of the trihalomethyl group with fluorine. Thus, for example, one mole of trichloromethylbenzoyl chloride may be reacted with three moles of fluorinating agent in the presence of a halogen transfer catalyst, to selectively fluorinate the methyl group. It is surprising that trifluoromethylbenzoyl chlorides or bromides are formed selectively and in high yield by the process of the invention, rather than a mixed product containing a statistical distribution of the fluorine atoms on the trihalomethyl group and the acid halide group. In addition to the fluorination that occurs as a result of reaction with the fluorinating agent, a halogen exchange occurs during the reaction, in the presence of the halogen transfer catalyst, that results in the selective distribution and/or re-distribution of the fluorine atoms.

Description of Preferred Embodiments

The preferred starting materials for the process of this invention are trichloromethylbenzoyl chloride characterized by the formula

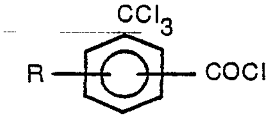

wherein R is hydrogen, chloro-, fluoro-, bromo-, nitro-, cyano- or methyl-; and most preferably, the compounds m-trichloromethylbenzoyl chloride and p-trichloromethylbenzoyl chloride. These compounds are reacted with three moles or less of hydrogen fluoride or trifluorobenzoyl fluoride in the presence of a halogen transfer catalyst, to prepare the corresponding trifluoromethylbenzoyl chlorides.

The fluorination reaction in accordance with the invention, is carried out in the presence of a halogen transfer catalyst. Such catalysts are well known in literature and include for example ferric chloride, aluminum chloride, molybdenum pentachloride, titanium tetrachloride, antimony pentafluoride, antimony pentachloride antimony-V-chloride-fluoride, and the like. The preferred catalyst is antimony pentachloride. Typically, the catalyst is employed in amounts of about 0.01 to about 10 percent by weight and preferably about 0.1 to about 3 percent by weight based on the weight of trichloromethylbenzoyl chloride starting material.

Under ideal stoichiometric conditions three moles of hydrogen fluoride or trifluoromethylbenzoyl fluoride may be contacted with one mole of trichloromethylbenzoyl chloride or tribromomethylbenzoyl bromide in the presence of a halogen transfer catalyst to yield four moles of trifluoromethylbenzoyl chloride or bromide. It will be understood however, that greater or less than stoichiometric amounts of the reactants can be employed. Thus, in accordance with the process of this invention substituted or unsubstituted trichloromethylbenzoyl chloride or tribromomethylbenzoyl bromide may be reacted with corresponding substituted or unsubstituted trifluoromethylbenzoyl fluorides, in the presence of a halogen transfer catalyst to produce the corresponding substituted or unsubstituted trifluoromethylbenzoyl chloride or bromide.

The process of the invention may be carried out over a wide range of conditions. The process is preferably carried out at atmospheric pressure, however subatmospheric and superatmospheric pressures may be employed if desired. The temperature range under atmospheric conditions may vary between the melting point and the boiling point of the reaction mixture. Preferably however the reaction is carried out in a temperature range from about 0 to about 100° Celcius and most preferably from about 50 to about 80° Celcius.

Although it is preferred to carry out the reaction neat, and suitable inert solvent may be employed if desired. Suitable solvents include for example, nitrobenzene, carbon disulfide, and the like.

The desired product, trifluoromethylbenzoyl chloride, is conveniently removed from the reaction product by conventional means such as fractional distillation. Under-fluorinated reaction products such as chloro-difluoromethyl-benzoyl chloride, dichlorofluoromethylbenzoyl chloride, and the like may be recycled for further reaction in accordance with the invention. Over fluorinated products such as trifluoromethylbenzoyl fluoride may be converted to trifluoromethylbenzoyl chloride by halogen exchange reaction in admixture with trichloromethylbenzoyl chloride in the presence of a halogen transfer catalyst.

It is a particular advantage of this invention that the desired product, such as trifluoromethyl benzoyl chloride, may be economically prepared from readily available, inexpensive starting materials in a simple, direct manner, requiring relatively few process steps. Thus, for example, xylene may be chlorinated in a known manner to prepare bis(trichloromethyl)benzene, which then may be readily hydrolyzed, for example by reaction with one mole of water, preferably in the presence of a catalyst, such as ferric chloride, to yield trichloromethylbenzoyl chloride. The trichloromethylbenzoyl chloride

may then be selectively fluorinated by reaction with about three moles of hydrogen fluoride in the presence of a halogen transfer catalyst to yield trifluoromethylbenzoyl chloride. Thus, in a simple direct method, requiring only three process steps, trifluoromethylbenzoyl chloride may be synthesized from a readily available commodity chemical, such as xylene. In addition, various other substituted xylenes, bearing ring substituents stable to the process steps may be employed as starting materials to produce correspondingly substituted trifluoromethylbenzoyl chlorides. Furthermore, it will be apparent that various other methylbenzene starting materials, such as mesitylene or durene, may be employed in a similar fashion to produce trifluoromethylbenzoyl chlorides having more than one trifluoromethyl and/or acyl chloride group present.

In a particular embodiment, the present invention provides, a process for the preparation of trifluoromethylbenzoyl chloride comprising the steps of (A) hydrolysing bis(trichloromethyl)benzene by reaction with about one mole of water to form trichloromethylbenzoyl chloride, and (B) reacting the trichloromethylbenzoyl chloride with about three moles or less of hydrogen fluoride, in the presence of halogen transfer catalyst, to yield trifluoromethylbenzoyl chloride.

The following specific examples are provided to further illustrate the invention. In the examples, unless otherwise indicated, all parts and percentages are by weight and all temperatures are in degrees Celcius.

### Example 1

A mixture of 2.3 parts of 3-trichloromethylbenzoyl chloride, 5.0 parts of 3-trifluoromethylbenzoyl fluoride, and 0.18 parts of antimony pentachloride was charged to a reaction vessel and heated to 75°—80°C. The mixture was maintained in that temperature range, with stirring, for about one hour, then cooled to room temperature and analyzed by gas chromatographic and mass spectroscopic techniques. The reaction product was found to contain about 22% 3-trifluoromethylbenzoyl fluoride; 56% 3-trifluoromethylbenzoyl chloride; 9% 3-trichloromethylbenzoyl chloride; and about 6% mixed fluorochloromethylbenzoyl halides.

### Example 2

A mixture of 2.9 parts of 4-trifluoromethylbenzoyl fluoride; 2.6 parts of 4-trichloromethylbenzoyl chloride and 0.04 parts of antimony pentachloride was charged to a reaction vessel and heated to about 55°C. The mixture was maintained at about that temperature, with stirring, for about six hours, then cooled to room temperature and analyzed by gas chromatographic and mass spectroscopic techniques. The reaction product was found to contain about 16.5% 4-trifluoromethylbenzoyl fluoride; 63.4% 4-trifluoromethylbenzoyl chloride; 15% mixed fluorochloromethylbenzoyl halides; 3.6% 4-trichloromethylbenzoyl chloride.

### Example 3

The procedure of Example 2 was repeated except that in place of the antimony pentachloride, there was substituted 0.04 parts of molybdenum pentachloride. Analysis of the reaction product by gas chromatography and mass spectroscopy indicated about 38% 4-trifluoromethylbenzoyl fluoride; 24% 4-trifluoromethylbenzoyl chloride; 18% mixed 4-trichloromethylbenzoyl halides; and 17% 4-trichloromethylbenzoyl chloride.

### Example 4

A mixture of 27 parts of 4-chlorodifluoromethylbenzoyl chloride, 23 parts of 4-dichlorofluoromethylbenzoyl fluoride, and 1.0 part of antimony pentachloride was charged to a reaction vessel and heated to about 55°C. The mixture was maintained at that temperature, with stirring, for about two hours, then cooled to room temperature. Analysis by gas chromatography and mass spectroscopy indicated a reaction product containing about 0.8% trifluoromethylbenzoyl fluoride; 39.7% trifluoromethylbenzoyl chloride; 36.3% chlorodifluoromethylbenzoyl chloride; and 15.4% dichlorofluoromethylbenzoyl chloride; and 5.9% trichloromethylbenzoyl chloride.

### Example 5

The procedure of Example 4 was repeated except that in place of the 4-chlorodifluoromethylbenzoyl chloride and 4-dichloromethylbenzoyl fluoride there was substituted 42 parts of 4-trichloromethylbenzoyl fluoride and 8 parts of 4-dichlorofluoromethylbenzoyl fluoride. The reaction product consisted of about 2% 4-trifluoromethylbenzoyl fluoride; 12.3% 4-trifluoromethylbenzoyl chloride; 22.1% 4-chlorodifluoromethylbenzoyl chloride; 22.3% 4-dichlorofluoromethylbenzoyl chloride; and 38.3% 4-trichloromethylbenzoyl chloride.

When the general process of the foregoing examples is repeated except that brominated reactants, such as tribromomethylbenzoyl bromide or fluoride, dibromofluoromethylbenzoyl bromide or fluoride, and the like are employed in substitution for the chlorinated reactants of the examples, a product containing trifluoromethylbenzoyl bromide is obtained. Similarly where various substituted trihalomethylbenzoyl halides having nuclear substituents $R_1$ and $R_2$ as defined hereinabove, are employed, the correspondingly substituted trifluoromethylbenzoyl chloride or bromides are obtained.

Example 6

A) A mixture of 626 parts of 1,4-bis(trichloromethyl)benzene and 3.1 parts of ferric chloride was charge to a reaction vessel equipped with a condenser. The mixture was heated and maintained at about 120°—130° while 34.2 parts of water was added, over a period of about 5 hours. The reaction product consisted of about 10% terephthaloyl chloride, about 74.2% 4-trichloromethylbenzoyl chloride and about 15.4% of 1,4-bis(trichloromethyl)benzene. The product was then fractionally distilled to recover essentially pure 4-trichloromethylbenzoyl chloride.

B) A mixture of 25.9 parts of the 4-trichloromethylbenzoyl chloride prepared as in Example 1A, above, and 0.26 parts of antimony pentachloride was charged to a reactor and heated to about 70°—75°C. The liquid was maintained at about 70°—75°C, with stirring, while HF vapor was bubbled into the bottom of the reactor over a period of 45 minutes until a total of about 2.2 parts of anhydrous HF has been added. The mixture was maintained at temperature, with stirring, for an additional 30 minutes, then cooled to about room temperature. The reaction product, based on gas chromatographic analysis was found to contain approximately 38% 4-trifluoromethylbenzoyl fluoride; 56% 4-trifluoromethylbenzoyl chloride; about 5% of mixed 4-fluorochloro-methylbenzoyl halides and less than 1.0% 4-trichloromethylbenzoyl chloride. The structure of the major product component, 4-trifluoromethylbenzoyl chloride was confirmed by spectral analyses.

Example 7

A mixture of about 103 parts of 4-trichloromethylbenzoyl chloride and 1.0 parts of antimony pentachloride was charged to a reactor and heated to 65°—70°C. The mixture was maintained at about that temperature with stirring over a 90 minute period while 16 parts of HF vapor was bubbled into the bottom of the reactor. The mixture was maintained at temperature, with stirring, for an additional hour, then cooled to about room temperature. The product analyzed by gas chromatographic techniques was found to contain about 35% 4-trifluoromethylbenzoyl fluoride; 55% 4-trifluoromethylbenzoyl chloride; less than 10% of mixed 4-fluorochloromethylbenzoyl halides and less than 1% 4-trichloromethylbenzoyl chloride.

Example 8

A quantity of 3-trichloromethylbenzoyl chloride was prepared by hydrolysis of 1,3-bis-(trichloromethyl)benzene with one mole of water in the presence of a catalytic amount of $FeCl_3$. A mixture of 9.2 parts of the 3-trichloromethylbenzoyl chloride and 0.1 parts of antimony pentachloride was charged to a reactor and heated to about 60°C. The liquid mixture was maintained at about 60°C, with stirring, while HF vapour was bubbled into the bottom of the reactor until a total of about 2.2 parts of anhydrous HF had been added. The mixture was maintained at temperature, with stirring, for an additional hour, then cooled to about room temperature. The product was analyzed by gas chromatography techniques as well as by spectral analyses. The major product was found to be 3-trifluoromethylbenzoyl chloride. The distribution of components in the reaction product as determined by gas chromatographic techniques, was approximately 18.4% 3-trifluoromethylbenzoyl fluoride; 52.0% 3-trifluoromethylbenzoyl chloride; less than 1% mixed 3-fluoro-chloromethyl benzoyl halides; and about 21% 3-trichloro methyl benzoyl choride.

When the general process of Examples 6B, 7, or 8 is repeated except that tribromomethylbenzoyl bromide is employed in substitution for the trichloromethylbenzoyl chloride starting material, a product containing trifluoromethylbenzoyl bromide as the major reaction product is obtained. Similarly, when various substituted trichloromethylbenzoyl chlorides or tribromomethylbenzoyl chlorides, having nuclear substituents $R_1$ and/or $R_2$ as defined hereinabove, are employed as the starting materials, the equivalent substituted trifluoromethylbenzoyl chlorides or trifluoromethylbenzoyl bromides are obtained.

## Claims

1. A process for the preparation of trifluoromethylbenzoyl halides of the formula

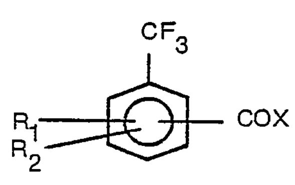

(I)

wherein X is chlorine, or bromine and $R_1$ and $R_2$ independently represent hydrogen, fluorine, chlorine, bromine, alkyl, aryl, haloaryl, alkoxy, fluoroalkoxy, aryloxy, haloaryloxy, nitro, cyano, sulfonyl or carboxylic acid chloride, characterised by reacting a compound of the formula

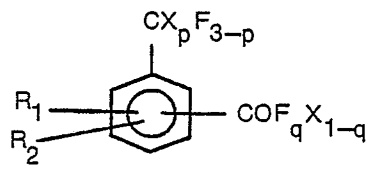

(II)

wherein p is 1, 2 or 3, q is 0 or 1 and X, $R_1$ and $R_2$ are as defined herein, with the fluorinating agent selected from hydrogen fluoride and a compound of the formula

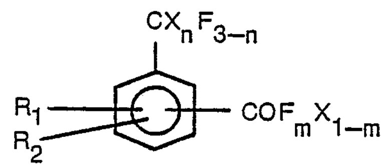

(III)

wherein n is 0, 1 or 2, m is 0 or 1, provided that when n is 0 m is 1, and X, $R_1$ and $R_2$ are as defined herein, said fluorinating agent being provided in an amount sufficient to replace the chlorine or bromine atom(s) represented by $X_p$ of formula (II) by fluorine, in the presence of a halogen transfer catalyst.

2. A process according to claim 1 when carried out at atmospheric pressure and at a temperature of 0° to 100° Celsius in the presence of antimony pentachloride as catalyst.

3. A process according to claim 1 or 2 wherein 4-trifluoromethylbenzoyl chloride is prepared by reacting 4-trichloromethylbenzoyl chloride (II) with 4-trifluoromethylbenzoyl fluoride (III).

4. A process according to claim 1 or 2 wherein 3-trifluoromethylbenzoyl chloride is prepared by reacting 3-trichloromethylbenzoyl chloride (II) with 3-trifluoromethylbenzoyl fluoride (III).

5. A process according to any one of the preceding claims wherein the amount of fluorinating agent is three moles or less per mole of the compound of formula (II).

6. A process according to claim 1 or 2 wherein 3-trifluoromethylbenzoyl chloride is prepared by reacting 3-trichloromethylbenzoyl chloride with three moles or less of hydrogen fluoride.

7. A process according claim 1 or 2 wherein 4-trifluoromethylbenzoyl chloride is prepared by reacting 4-trichloromethylbenzoyl chloride with three moles or less of hydrogen fluoride.

8. A process for the preparation of trifluoromethylbenzoyl chlorides characterized by the steps of A) hydrolyzing bis(trichloromethyl)benzene with about one mole of water to form trichloromethylbenzoyl chloride, and B) reacting the trichloromethyl benzoyl chloride with three moles or less of hydrogen fluoride, in the presence of a halogen transfer catalyst.

**Patentansprüche**

1. Verfharen zur Herstellung von Trifluormethylbenzoyl-halogeniden der Formel I

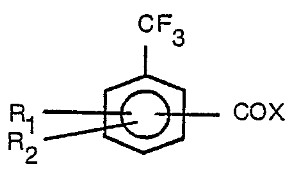

(I)

worin X für Chlor oder Brom steht und $R_1$ und $R_2$ unabhängig Wasserstoff, Fluor, Chlor, Brom, Alkyl, Aryl, Halogenaryl Alkoxy, Fluoralkoxy, Aryloxy, Halogenaryloxy, Nitro, Cyano, Sulfonyl oder Carbonsäurechlorid bedeuten, dadurch gekennzeichnet, daß eine Verbindung der Formel II

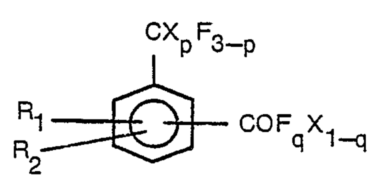

(II)

worin p gleich 1, 2 oder 3 ist, q 0 oder 1 bedeutet und X, $R_1$ und $R_2$ die oben genannte Bedeutung haben, mit einem Fluorierungsmittel, ausgewählt unter Fluorwasserstoff und einer Verbindung der Formel III

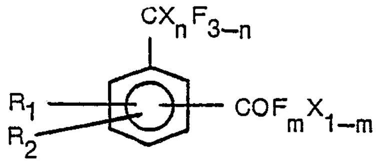

(III)

worin n gleich 0, 1 oder 2 ist, m für 0 oder 1 steht, mit der Maßgabe, daß wen n gleich 0 ist, m für 1 steht, und X, $R_1$ und $R_2$ die genannte Bedeutung haben, wobei das Fluorierungsmittel in einer Menge zur Verfügung gestellt wird, die hinreichend ist, um die Chlor- oder Bromatome, die durch $X_p$ in Formel II dargestellt werden, durch Fluor zu ersetzen, in Gegenwart eines Halogentransferkatalysators umgesetzt wird.

2. Verfahren nach Anspruch 1, dadurch gekennzeichnet, daß es unter atmosphärischem Druck und bei einer Temperatur von 0 bis 100°C in Gegenwart von Antimonpentachlorid als Katalysator durchgeführt wird.

3. Verfahren nach Anspruch 1 oder Anspruch 2, worin 4-Trifluormethylbenzoylchlorid durch Umsetzung von 4-Trichlormethylbenzoylchlorid (II) mit 4-Trifluormethylbenzoylfluorid (III) hergestellt wird.

4. Verfahren nach Anspruch 1 oder 2, worin 3-Trifluormethylbenzoylchlorid durch Umsetzung von 3-Trichlormethylbenzoylchlorid (II) mit 3-Trifluormethylbenzoylfluorid (III) hergestellt wird.

5. Verfahren nach einem der voranstehenden Ansprüche, worin die Menge des Fluorierungsmittels drei Mole oder weniger pro Mol der Verbindung nach Formel II beträgt.

6. Verfahren nach Anspruch 1 oder Anspruch 2, worin 3-Trifluormethylbenzoylchlorid durch Umsetzung von 3-Trichlormethylbenzoylchlorid mit drei Molen oder weniger Fluorwasserstoff hergestellt wird.

7. Verfahren nach Anspruch 1 oder 2, worin 4-Triflourmethylbenzoylchlorid durch Umsetzung von 4-Trichlormethylbenzoylchlorid mit drei Molen oder weniger Fluorwasserstoff hergestellt wird.

8. Verfahren zur Herstellung von Trifluormethylbenzoylchloriden, gekennzeichnet durch A) Hydrolysieren von Bis(trichlormethyl)benzol mit etwa einem Mol Wasser, um Trichlormethylbenzoylchlorid zur bilden und B) Umsetzung des Trichlormethylbenzoylchlorids mit drei Molen oder weniger Fluorwasserstoff in Gegenwart eines Halogentransferkatalysators.

## Revendications

1. Procédé pour la préparation d'halogénures de trifluorométhylbenzoyle de la formule:

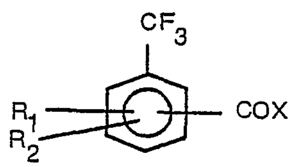

(I)

dans laquelle X est du chlore ou du brome et $R_1$ et $R_2$ représentent indépendamment de l'hydrogène, du fluor, du chlore, du brome, un radical alkyle, aryle, haloaryle, alkoxy, fluoroalkoxy, aryloxy, haloaryloxy, nitro, cyano ou sulfonyle ou un chlorure d'acide carboxylique, caractérisé en ce qu'on fait réagir un composé de la formule:

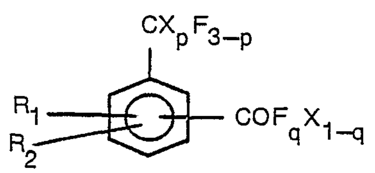

(II)

dans laquelle p est égal à 1, 2 ou 3, q est égal à 0 ou 1, et X, $R_1$ et $R_2$ sont comme définis ci-dessus, avec un agent de fluoration choisi parmi le fluorure d'hydrogène et un composé de la formule:

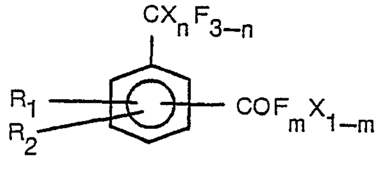

(III)

dans laquelle n est égal à 0, 1 ou 2, m est égal à 0 ou 1, pourvu que, lorsque n est égal à 0, m est égal à 1, et X, $R_1$ et $R_2$ sont comme définis ci-dessus, la quantité de cet agent de fluoration étant suffisant pour

remplacer le ou les atomes de chlore ou de brome représentés par $X_p$ de la formule (II) par du fluor, en la présence d'un catalyseur de transfert d'halogène.

2. Procédé selon la revendication 1, conduit à la pression atmosphérique et à la température de 0 à 100°C Celsius en la présence de pentachlorure d'antimoine come catalyseur.

3. Procédé selon la revendication 1 ou la revendication 2, dans lequel on prépare un chlorure de 4-trifluorométhylbenzoyle en faisant réagir le chlorure de 4-trichlorométhylbenzoyle (II) avec du fluorure de 4-trifluorométhylbenzoyle (III).

4. Procédé selon la revendication 1 ou la revendication 2, dans lequel on prépare le chlorure de 3-trifluorométhylebenzoyle en faisant réagir le chlorure de 3-trichlorométhylbenzoyle (II) avec le fluorure de 3-trifluorométhylbenzoyle (III).

5. Procédé l'une des revendications précédentes, dans lequel la quantité d'agent de fluoration est 3 moles ou moins par mole du composé de la formule (II).

6. Procédé selon la revendication 1 ou la revendication 2, dans lequel le chlorure de 3-trifluorométhylbenzoyle est préparé en faisant réagir le chlorure de 3-trichlorométhylbenzoyle avec 3 moles ou moins de fluorure d'hydrogène.

7. Procédé selon la revendication 1 ou la revendication 2, dans lequel le chlorure de 4-trifluorométhylbenzoyle est préparé en faisant réagir le chlorure de 4-trichlorométhylebenzoyle avec 3 moles ou moins de fluorure d'hydrogène.

8. Procédé pour la préparation de chlorures de trifluorométhylbenzoyle, caractérisé par les étapes suivantes: A) hydrolyser le bis(trichlorométhyle)benzène avec environ 1 mole d'eau pour former du chlorure de trichlorométhylbenzoyle, et B) faire réagir le chlorure de trichlorométhylbenzoyle avec 3 moles ou moins de fluorure d'hydrogène, en la présence d'un catalyseur de transfert d'halogène.